# EUROPEAN PATENT SPECIFICATION

(11) **EP 4 017 989 B1**
(45) Date of publication and mention of the grant of the patent: **18.12.2024**
(21) Application number: 20853637.5
(22) Date of filing: 20.08.2020
(51) Int. Cl.: C12P 19/34, C12Q 1/6806, C12Q 1/6869, C12Q 1/6853

(54) **HAIRPIN PRIMER DESIGN FOR SEQUENTIAL PCR PRODUCTION OF TARGETED SEQUENCING LIBRARIES**
HAIRPIN-PRIMER-ENTWURF ZUR SEQUENZIELLEN PCR-PRODUKTION VON ZIELGERICHTETEN SEQUENZIERUNGSBIBLIOTHEKEN
CONCEPTION D'AMORCE EN ÉPINGLE À CHEVEUX POUR LA PRODUCTION DE PCR SÉQUENTIELLE DE BIBLIOTHÈQUES DE SÉQUENÇAGE CIBLÉES

(30) Priority: 20.08.2019 US 201962889105 P
(43) Date of publication of application: 29.06.2022
(73) Proprietor: ILLUMINA, INC., San Diego, CA 92122 (US)
(72) Inventor: MELTZER, Robert, Belmont, MA 02478 (US); FONTANEZ, Kristina, Arlington, MA 02476 (US); SCHENK, Desiree, Watertown, MA 02472 (US)
(74) Representative: Graham Watt & Co LLP
(86) International application number: PCT/US2020/047214
(87) International publication number: WO 2021/035056

(56) References cited:
- WO-A1-2015/157369
- WO-A1-2016/025815
- WO-A1-2016/138080
- WO-A1-2016/138080
- WO-A1-2017/161306
- WO-A1-2018/175399
- WO-A1-2019/140298

## Description

### INTRODUCTION

With the advent and adoption of massive parallel sequencing (such as next generation sequencing), investigators interrogate a large swath of nucleic acid information from samples, such as DNA or RNA samples. In many cases, investigating the entire genome or large portions of it (exome sequencing) is cost-prohibitive, requires large-capacity sequencing, and produces large amounts of data beyond what may be necessary for clinical settings. Therefore, targeted sequencing for specific applications allows for a focused method to interrogate relevant nucleic acid regions of interest. A prevalent application is targeted sequencing for oncology, which provides specific mutational information from a biopsy sample which may be used to guide treatment or monitor treatment response. For such applications, nucleic acids are extracted from a sample, such as a biological sample, and enriched to prepare DNA libraries for sequencing, wherein a library is a set of DNA molecules prepared from a particular sample after the enrichment process. For efficient and cost-effective enrichment, the enrichment of multiple targeted genomic sequences is performed in parallel (multiplexed). Without multiplexing, a sample must undergo multiple enrichment steps, which can be burdensome with samples of low quantity, not to mention the multiplied cost of reagents, processing time, and reduced sample throughput.

WO 2019/140298 relates to a loopable primer, the split primer and spli-loopable primer and uses thereof.

WO 2018/175399 relates to methods of target nucleic acid amplification though the use of a universal hairpin primer and uses thereof.

WO 2016/138080 relates to methods for the detection, identification and quantification of target nucleic acids in a sample using hairpin barcode primers to incorporate unique barcodes into target nucleic acids in a PCR pre-amplification step.

The present disclosure provides an approach for simultaneous construction of targeted sequencing libraries for multiple targeted genomic sequences within a single sample and related advantages.

### SUMMARY

The invention is defined by the appended claims. The methods and compositions described herein provide a method of library preparation and a composition suitable for nucleic amplification by that method.

The hairpin primer may comprise:
(a) a target-specific primer sequence comprising a nucleotide sequence complementary to a portion of the nucleotide sequence of the target nucleic acid;
(b) an adaptor sequence;
(c) a lock sequence comprising a sequence complementary to the said target-specific primer sequence;
wherein sequences (a) to (c) are arranged from the 3' end to the 5' end of the said hairpin primer. The target-specific primer sequence (a) and complementary lock sequence (c) are able to hybridize, thus allow the said hairpin primer to form a secondary hairpin structure. The target- specific primer sequence and lock sequence, when hybridized, form the stem portion of the said hairpin structure.

In some aspects of the hairpin primer, the adapter sequence comprises a universal primer sequence.

In some aspects of the hairpin primer, the lock sequence is complementary to a portion, or all, of the target-specific primer sequence.

In some aspects, the hairpin primer further comprises a gene-specific hairpin de-stabilizer sequence located 5' to the target-specific primer sequence, wherein the gene-specific hairpin de- stabilizer sequence comprises of at least one nucleotide that is non-complementary to the sequence upstream of the portion of the target nucleic acid sequence complementary to target- specific primer sequence.

In some aspects, the hairpin primer further comprises a semi-random sequence, referred to herein as Molecular Identifier. The Molecular Identifier (MI) sequence is located 5' to the gene-specific hairpin de-stabilizer sequence. In an embodiment of the presently disclosed hairpin primer, the MI sequence does not contain long GC (guanine and cytosine bases) stretches. This serves to avoid, or reduce, mis-pairing with other GC-rich sequences at the annealing temperature (discussed below). In some aspects, the MI sequence is a semi-random 9-mer, and wherein positions 1, 4, and 7 are restricted to either ATP or TTP. In other aspects, the MI sequence is longer and may be in the range of about 9-15-mer.

In some aspects, the hairpin primer further comprises an adaptor hairpin de-stabilizer sequence located 5' to the adaptor sequence, wherein the adaptor hairpin de-stabilizer sequence comprises at least one nucleotide derived from the gene-specific portion of the hairpin primer, wherein said one nucleotide is non-complementary to the first nucleotide of the hairpin primer that does not participate in the hairpin stem.

In some aspects, the hairpin primer further comprises a stem de-stabilizer sequence located 5' to the lock sequence, wherein the stem de-stabilizer sequence comprises at least one nucleotide which is non-complementary to the 3' portion of the target-specific primer sequence of the hairpin primer.

In some aspects, the secondary hairpin structure of the hairpin primer as described in any of the previous aspects is denatured at a temperature in the range of about 55-80°C. More specifically, the secondary hairpin structure of the hairpin primer as described in any of the previous aspects is denatured at a temperature in the range of about 62-72°C.

In some aspects, the secondary hairpin structure of the hairpin primer as described in any of the previous aspects is denatured at a temperature of 62°C.

In some aspects, the secondary hairpin structure of the hairpin primer as described in any of the previous aspects is denatured at a temperature of 72°C.

The present disclosure provides a method of amplifying target nucleic acid, the method comprising the steps of:
(a) providing a single reaction mixture comprising:
   (i) DNA sample;
   (ii) the hairpin primer of any one of claims 1-13;
   (iii) a universal primer comprising all, or some, of the adaptor sequence of the hairpin primer of (ii);
   (iv) amplification reagents; and
   (iv) a DNA polymerase;
(b) subjecting the sample DNA to DNA amplification wherein the hairpin primer anneals to target sequences to allow for production of a target-specific amplification product; and
(c) subjecting the target-specific amplification product of step (b) to DNA amplification wherein the universal primer anneals to the said amplification product to allow for universal amplification of the products of step (b).

In some aspects of the method of amplifying target nucleic acid, the DNA sample is derived from a biological sample, for example, genomic DNA

In some aspects of the method of amplifying target nucleic acid, the DNA polymerase is selected from Taq DNA polymerase, Phusion polymerase, Platinum SuperFi, or Q5 polymerase. However, any polymerase which lack strand displacement activity is suitable for the presently described methods. In some aspects of the method of amplifying target nucleic acid, the DNA amplification of step (b) is preceded by a DNA denaturing incubation. In some aspects, the DNA denaturing incubation is performed at 98°C for two minutes.

In some aspects of the method of amplifying target nucleic acid, the DNA amplification of step (b) comprises the steps of denaturing the DNA sample; annealing the hairpin primer with the DNA to allow the formation of a DNA-primer hybrid; and incubating the DNA-primer hybrid to allow the DNA polymerase to synthesize an amplification product. In some aspects, the DNA amplification of step (b) is repeated at least two times.

In some aspects of the method of amplifying target nucleic acid, the annealing and DNA synthesis steps of the DNA amplification of step (b) are performed at a temperature range of about 55-80°C. In some aspects of the method of amplifying target nucleic acid, the annealing and DNA synthesis steps of the DNA amplification of step (b) are performed at a temperature range of about 62-72°C. In some aspects, the annealing and DNA synthesis steps of the DNA amplification of step (b) are performed at a temperature of 62°C. In other aspects, the annealing and DNA synthesis steps of the DNA amplification of step (b) are performed at a temperature of 72°C.

In some aspects of the method of amplifying target nucleic acid, the DNA amplification of step (c) comprises the steps of denaturing the DNA comprising amplification product of step (b); annealing the universal primer with the amplification product to allow the formation of a DNA-primer hybrid; and incubating the DNA-primer hybrid to allow the DNA polymerase to synthesize a second amplification product. In some aspects, the DNA amplification of step (c) is repeated at least twenty times. In other aspects, the DNA amplification of step (c) is repeated at least thirty times.

In some aspects of the method of amplifying target nucleic acid, the annealing and DNA synthesis steps of the DNA amplification of step (c) are performed at a temperature lower than the temperature used for the annealing and DNA synthesis steps of step (b).

In some aspects of the method of amplifying target nucleic acid, the temperature used for the annealing and DNA synthesis steps of the DNA amplification of step (c) is lower than the temperature required for the denaturation of the hairpin secondary structure of the hairpin primer, thus preventing, or reducing the likelihood of, the hybridization of the hairpin primer to the amplification products of step (b), sample DNA, or the universal primer sequence of the universal primers.

In some aspects of the method of amplifying target nucleic acid, the annealing and DNA synthesis steps of the DNA amplification of step (c) are performed at a temperature in the range of about 55-62°C. In some some aspects of the method of amplifying target nucleic acid, the annealing and DNA synthesis steps of the DNA amplification of step (c) are performed at a temperature of 62°C.

In some aspects of the method of amplifying target nucleic acid, the method of amplifying target nucleic acid further comprises a transition DNA amplification step performed after step (b) and before step (c). In some aspects, the transition DNA amplification step comprises the steps of denaturing the DNA comprising the amplification product of step (b); annealing the universal primer or the hairpin primer with the said amplification product to allow the formation of a DNA-primer hybrid; and incubating the DNA-primer hybrid to allow the DNA polymerase to synthesize an amplification product. In some aspects, the transition DNA amplification step is repeated at least two times.

In some aspects of the method of amplifying target nucleic acid, the annealing and DNA synthesis steps of the transition DNA amplification step are performed at a temperature lower than the temperature used for the annealing and DNA synthesis steps of step (b), but higher than the temperature used for the annealing and DNA synthesis steps of step (c).

In some aspects of the method of amplifying target nucleic acid, the annealing and DNA synthesis steps of the transition DNA amplification step are performed at a temperature in the range between the temperature used for the annealing and DNA synthesis steps of step (b), and the temperature used for the annealing and DNA synthesis steps of step (c).

In some aspects of the method of amplifying target nucleic acid, the annealing and DNA synthesis steps of the transition DNA amplification step are performed at a temperature in the range between the temperature used for the annealing and DNA synthesis steps of step (b), and the temperature used for the annealing and DNA synthesis steps of step (c), and wherein the temperature of annealing and DNA synthesis steps of the transition DNA amplification step drops gradually with every repeat. In some aspects, the likelihood of hybridization of the hairpin primer to the amplification products of step (b) is reduced with every repeat. In some aspects, the annealing and DNA synthesis steps of the transition DNA amplification step are performed at the first repeat at a temperature of 72°C, at a second repeat at a temperature of 70°C, at a third repeat at a temperature of 68°C, at a fourth repeat at a temperature of 66°C, at a fifth repeat at a temperature of 64°C, and at a sixth repeat at a temperature of 62°C. The amplification method described in the present paragraph is also known in the art as "touchdown PCR".

In some aspects of the method of amplifying target nucleic acid, the universal primer further comprises an application adaptor sequence. In some aspects, the application adaptor sequence may be an indexing sequence, a barcode sequence, a tag for the amplification products detection, purification or quantification, or a sequencing adaptor for sequencing applications. In some aspects of the method of amplifying target nucleic acid, the universal primer for amplifying the amplification products is Universal 500.F, and the universal primer for amplifying the amplification products reverse complement is Universal 700.R.

In any one of the aspects of the method of amplifying target nucleic acid, the amplification products may be used to prepare a targeted sequencing library.

In an aspect, provided herein is a method of amplifying a target nucleic acid molecule, comprising: providing a sample comprising the target nucleic acid molecule and a hairpin primer for amplifying the target nucleic acid molecule or reverse complement thereof, wherein the hairpin primer comprises (i) a target-specific primer sequence comprising a nucleic acid sequence complementary to a portion of a nucleic acid sequence of the target nucleic acid molecule; (ii) an adaptor sequence; (iii) a lock sequence comprising a nucleic acid sequence complementary to the target-specific primer sequence; wherein (i) to (iii) are arranged from a 3' end to a 5' end of the hairpin primer; wherein the target-specific primer sequence and the lock sequence are able to hybridize, thereby allowing the hairpin primer to form a hairpin structure, and wherein the target-specific primer sequence and the lock sequence, when hybridized, form a stem portion of the hairpin structure.

In some aspects, the portion of the nucleic acid sequence of the target nucleic acid molecule hybridizes to the target-specific primer sequence of the hairpin primer.

In some aspects, the sample further comprises a universal primer comprising at least a portion of the adaptor sequence of the hairpin primer.

In some aspects, the sample further comprises an amplification reagent. In some aspects, the sample further comprises a DNA polymerase.

In some aspects, the method further comprises subjecting the sample to an amplification condition, wherein the hairpin primer is extended using the target nucleic acid sequence as a template to produce an amplification product.

In some aspects, the method further comprises subjecting the sample to an additional amplification condition, wherein the universal primer anneals to the amplification product to allow for universal amplification of the amplification product.

In some aspects, the amplification condition comprises a temperature for performing an annealing step and a DNA synthesis step in a range of about 55-80°C.

In some aspects, the amplification condition comprises a temperature for performing an annealing step and a DNA synthesis step in a range of about 62-72°C.

In some aspects, the temperature is about 62°C.

In some aspects, the temperature is about 72°C.

In some aspects, the additional amplification condition comprises a temperature for an annealing step and a DNA synthesis step in a range of about 50-80°C.

In some aspects, the additional amplification condition comprises a temperature for an annealing step and a DNA synthesis step in a range of about 60-62°C.

In some aspects, the temperature is 62°C.

In some aspects, the method further comprises subjecting the sample to a transition amplification step performed after the amplification condition and before the additional amplification condition.

In some aspects, the transition amplification condition comprises denaturing the amplification product; annealing the universal primer or the hairpin primer with the amplification product to allow formation of an amplification product-primer hybrid; and incubating the amplification product-primer hybrid to allow synthesis of an additional amplification product.

In as aspect, provided herein is a droplet comprising a target nucleic acid molecule and a hairpin primer for amplifying the target nucleic acid molecule or reverse complement thereof, wherein the hairpin primer comprises (i) a target-specific primer sequence comprising a nucleic acid sequence complementary to a portion of a nucleic acid sequence of the target nucleic acid molecule; (ii) an adaptor sequence; (iii) a lock sequence comprising a nucleic acid sequence complementary to the target-specific primer sequence; wherein (i) to (iii) are arranged from a 3' end to a 5' end of the hairpin primer; wherein the target-specific primer sequence and the lock sequence are able to hybridize, thereby allowing the hairpin primer to form a hairpin structure, and wherein the target-specific primer sequence and the lock sequence, when hybridized, form a stem portion of the hairpin structure.

In some aspects, the droplet is a particle.

In some aspects, the particle is a bead.

In as aspect, provided herein is a method of amplifying a target nucleic acid molecule in a droplet, comprising: generating a plurality of droplets, each droplet of the plurality comprising a target nucleic acid and a hairpin primer, wherein the hairpin primer comprises (i) a target- specific primer sequence comprising a nucleic acid sequence complementary to a portion of a nucleic acid sequence of the target nucleic acid molecule; (ii) an adaptor sequence; (iii) a lock sequence comprising a nucleic acid sequence complementary to the target-specific primer sequence; wherein (i) to (iii) are arranged from a 3' end to a 5' end of the hairpin primer; wherein the target-specific primer sequence and the lock sequence are able to hybridize, thereby allowing the hairpin primer to form a hairpin structure, and wherein the target-specific primer sequence and the lock sequence, when hybridized, form a stem portion of the hairpin structure.

In some aspects, the portion of the nucleic acid sequence of the target nucleic acid molecule hybridizes to the target-specific primer sequence of the hairpin primer.

In some aspects, the droplet further comprises a universal primer comprising at least a portion of the adaptor sequence of the hairpin primer.

In some aspects, the droplet further comprises an amplification reagent.

In some aspects, the droplet further comprises a DNA polymerase.

In some aspects, the method further comprises subjecting the droplet to an amplification condition, wherein the hairpin primer is extended using the target nucleic acid sequence as a template to produce an amplification product.

In some aspects, the method further comprises subjecting the droplet to an additional amplification condition, wherein the universal primer anneals to the amplification product to allow for universal amplification of the amplification product.

In some aspects, the amplification condition comprises a temperature for performing an annealing step and a DNA synthesis step in a range of about 55-80°C. In some aspects, the amplification condition comprises a temperature for performing an annealing step and a DNA synthesis step in a range of about 62-72°C.

In some aspects, the temperature is about 62°C.

In some aspects, the temperature is about 72°C.

In some aspects, the additional amplification condition comprises a temperature for an annealing step and a DNA synthesis step in a range of about 50-80°C.

In some aspects, the additional amplification condition comprises a temperature for an annealing step and a DNA synthesis step in a range of about 60-62°C. In some aspects, the temperature is 62°C.

In some aspects, the method further comprises subjecting the droplet to a transition amplification step performed after the amplification condition and before the additional amplification condition. In some aspects, the transition amplification condition comprises denaturing the amplification product; annealing the universal primer or the hairpin primer with the amplification product to allow formation of an amplification product-primer hybrid; and incubating the amplification product-primer hybrid to allow synthesis of an additional amplification product.

In some aspects, the droplet further comprises a particle. In some aspects, the particle is a bead.
In some aspects, the droplet is generated by shaking, vortexing, or a microfluidic device.

### BRIEF DESCRIPTION OF THE DRAWINGS

The invention may be best understood from the following detailed description when read in conjunction with the accompanying drawings. Included in the drawings are the following figures:

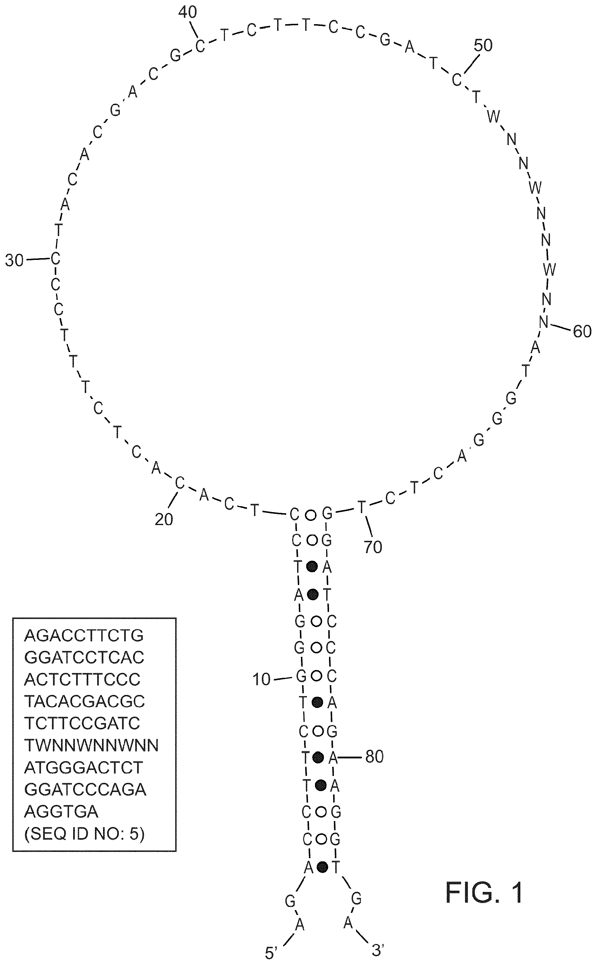

FIG. 1 is a schematic of a hairpin secondary structure of an embodiment of a hairpin primer, depicting its nucleotide sequence ("G" represents guanine, "C" represents cytosine, "A" represents adenine, and "T" represents thymine). Base pairing in the stem portion of the hairpin structure is schematically shown by open (guanine-cytosine) and solid (adenine-thymine) dots.

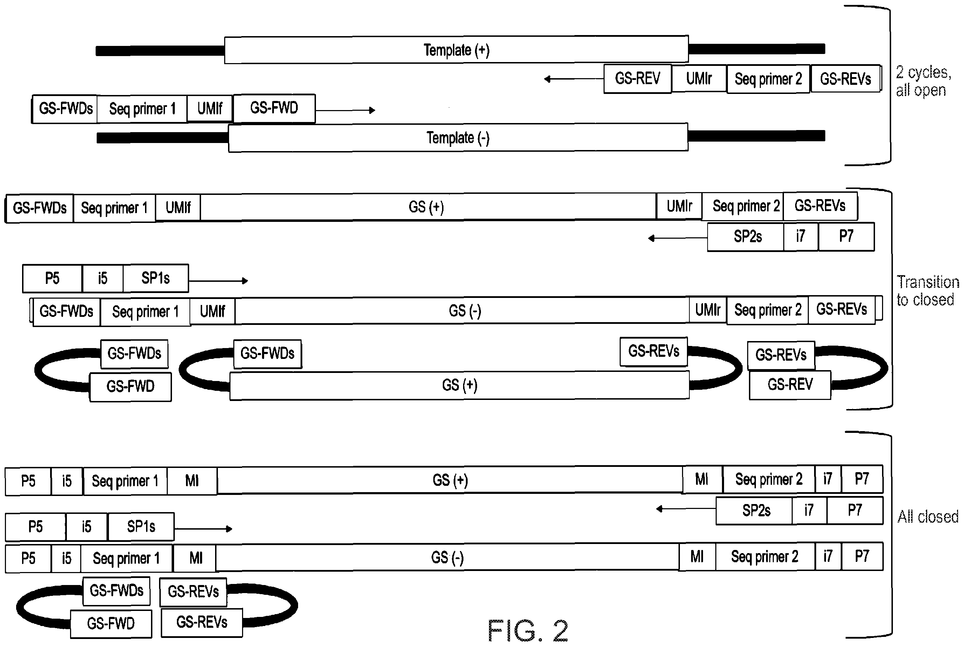

FIG. 2 is a schematic illustration depicting an embodiment of phases of thermal cycling amplification reaction (such as PCR) using an embodiment of the hairpin and universal primers of the present disclosure. Sample nucleic acids are depicted as two-dimensional elongated bar- shaped forms, wherein the target sequences are depicted as thicker portions of the bars. The sample DNA is featured in a denatured state wherein the 5' to 3' target strand (sense) is marked "Template(+)", and its reverse complement (antisense) is marked "Template(-)". The depicted hairpin primers comprise a target-specific primer sequence. In the forward hairpin primer, which primes Template(-), the target-specific primer sequence is marked "GS-FWD", while in the reverse hairpin primer, which prime Template (+), the target-specific primer sequence is marked "GS-REV". The target-specific primer sequence hybridizes (base-pairs) with complementary sequences (also referred to herein as "regions") of the target nucleic acids, for example the box marked "GS-FWD" base-pairs with a sequence in Template(-). The depicted forward hairpin primer also contains an MI ("MIf'), adaptor ("Seq. primer I"), and lock ("GS-FWDs"). The depicted reverse hairpin primer also contains an MI ("MIr"), adaptor ("Seq. primer 2"), and lock ("GS-REVs"). Arrows mark the direction of DNA synthesis. Amplicons (also referred to herein as "amplification products") created of Template(+) are marked "GS(+)", while amplicons created of Template(-) are marked "GS(-)". Hairpin primers are also illustrated in a secondary hairpin structure form, wherein the loop portion of the hairpin is represented by a curved line (the loop comprises the MI and adaptor portions of the hairpin primer) which connects the target- specific primer sequences and the locks which are illustrated forming the stem structure of the hairpin by base-pairing. The universal primers illustrated contain universal adaptors "SP2s" or "SPls", which represent universal adaptor sequences comprising all, or a portion, of the adaptor sequences of the hairpin primers used in the gene-specific amplification phase of the same amplification reaction. Therefore, the universal primers can hybridize with GS (+) and GS (-), and prime DNA synthesis. The universal primers are illustrated containing also applications adaptors "i7" and "P7" or "PS" and "i5", which are useful in later applications. The resulting amplification products illustrated in the "universal amplification" phase include the target sequences of interest, Mis, adaptors, and application adaptors.

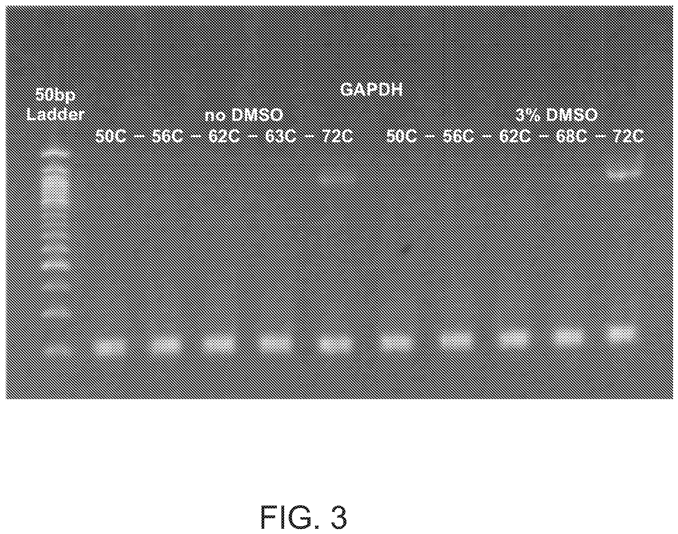

FIG. 3 is a picture of an agarose gel featuring the amplification products of the method of Example 1. Amplification of a portion of the GAPDH gene was performed using only target- specific hairpin primers. At lower temperatures, the hairpin structure of the hairpin primers prevents the hairpin primers from priming an amplification reaction. In the first five lanes after the ladder, amplification took place without the presence of DMSO, wherein in subsequent lanes DMSO was included. The presence of DMSO helps denature the secondary hairpin structure, especially when the annealing/extension steps are performed at 72°C.

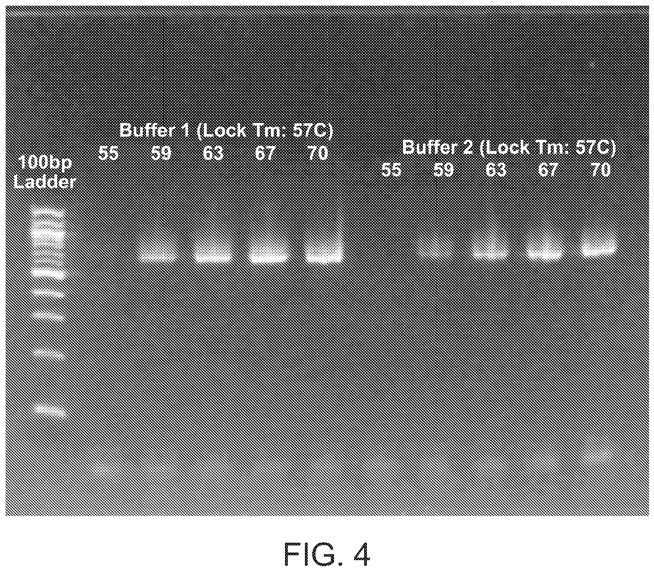

FIG. 4 is a picture of an agarose gel featuring the amplification products of the method of Example 2. Amplification reactions of a portion of the GAPDH gene was performed in two buffer systems, Buffer I and Buffer 2. Amplification product is observed at all temperatures used for the annealing/extension steps, except for 55°C, which is not high enough to denature the hairpin secondary structure. The amount of amplification product is increased with an increased annealing/extension temperature, indicating that the hairpins structures are only partially denatured at 59°C, particularly in the second buffer system. At 67°C and 70°C, the amplification product yield has improved over that of the reaction at 59°C (for both buffers). The hairpins were designed such that their melting temperatures were 57°C (50% of molecules denatured) and would therefore be fully denatured at 67°C and 70°C.

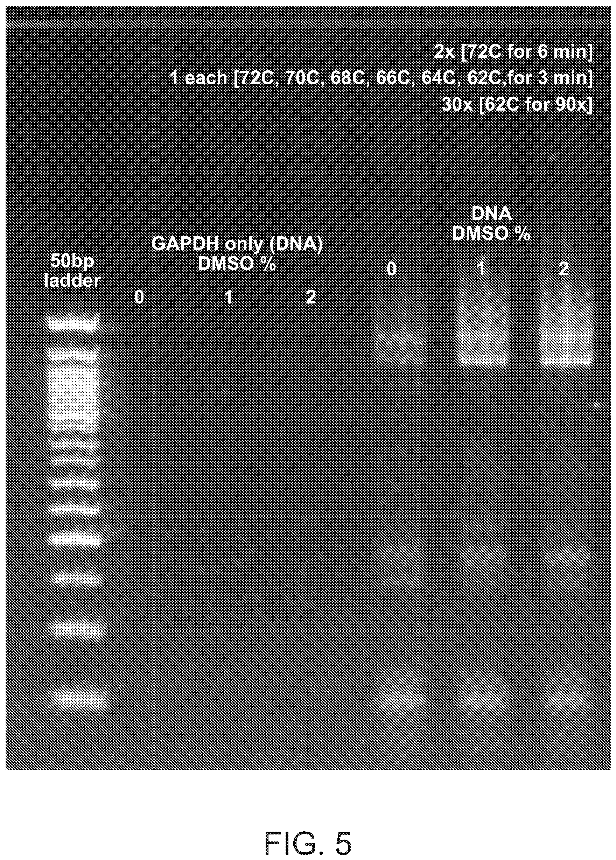

FIG. 5 is a picture of an agarose gel featuring the amplification products of the method of Example 3 which describes an embodiment of the method for creating GAPDH-specific sequencing libraries. PCR amplification was performed without the presence of universal oligonucleotides in the reaction mixture, and in the presence of 0%, I%, and 2% of DMSO (first three lanes after the ladder). The next three lanes show the amplification products of similar amplification reactions, however in these reactions universal oligos were also included in the amplification reaction. When only the target-specific hairpin primers are present in the amplification reaction, no product is detected in the completion of the reaction, indicating that the GAPDH-specific amplicons are created, or greatly increased in quantity, by the presence of universal primers.

### DETAILED DESCRIPTION

The present disclosure provides methods of hairpin primer design and targeted amplification thereof for creation of targeted sequencing libraries. Generally, the present methods allow for simultaneous construction of targeted sequencing libraries for multiple targeted nucleic acid sequences within a single sample. More specifically, the presently disclosed methods generate efficient and targeted sequence amplification while avoiding non-specific interaction of multiplex primers, avoiding non-specific amplification of sequences due to random priming from short sequences or molecular "tags" (such as Molecular Identifiers ("MI") or barcodes sequences), and avoiding unintentional interactions between target specific (also referred to herein as "gene specific") and universal primers during universal PCR amplification.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and exemplary methods and materials are now described. It must be noted that as used herein and in the appended claims, the singular forms "a," "an," and "the" include plural referents unless the context clearly dictates otherwise.

Thus, for example, reference to "a primer" includes a plurality of such primers, "target nucleic acid" includes a plurality of such targets, and reference to "the nucleic acid" includes reference to one or more nucleic acids and equivalents thereof known to those skilled in the art, and so forth.

It is further noted that the claims may be drafted to exclude any element which may be optional. As such, this statement is intended to serve as antecedent basis for use of such exclusive terminology as "solely", "only" and the like in connection with the recitation of claim elements, or the use of a "negative" limitation.

The publications discussed herein are provided solely for their disclosure prior to the filing date of the present application. Further, the dates of publication provided may be different from the actual publication dates which may need to be independently confirmed.

Any recited method can be carried out in the order of events recited or in any other order which is logically possible. For example, described herein are a variety of additional methods and applications, which may be performed in connection with the methods described herein relating to the hairpin primer design for amplification of target nucleic acids.

### Methods

The present invention describes methods for simultaneous (one tube reaction) construction of targeted sequencing libraries for multiple targeted nucleic acid sequences within a single nucleic acids sample (such as DNA sample). Targeted sequencing for specific applications allows for a focused method to interrogate relevant regions (such as DNA sequences) of interest.

Targeted library preparation methods generally consist of two categories: hybrid capture and amplification-based library preparation. Both methods first require selection of target sequences using selective primers. The former method uses the specific (selective) primers (also known as probes or "bait") to pull out the targeted regions from the rest of the sample. The latter relies on polymerase chain reaction (PCR) to amplify the targeted regions exponentially. With the hybrid capture approach, the isolated sequences also require adaptor sequences to be appended for compatibility with next generation sequencing (massive parallel sequencing). This is usually done via ligation or a separate amplification, adding additional processing time to the library preparation. Hybrid capture methods usually require larger sample quantities than amplification- based libraries with multiple, longer steps to prepare the sample. Amplification reactions are shorter in duration with less input, but as PCR introduces error, hybrid capture has a performance edge over amplification for very high multiplexing. Therefore, reducing library preparation to a single, easy-setup amplification reaction drives down the preparation time, reduces steps in which to introduce error, and increases throughput for investigators with multiple samples.
For amplification-based, targeted library preparation, previous attempts to generate multiplex sequencing libraries suffer from several limitations. To name some, to prepare large, multiplexed libraries, large numbers of primers are pooled together. This often results in little to no amplification product due to primer dimers from inter-primer interactions which can completely overwhelm an amplification reaction because of their increased amplification efficiency (driven by the high concentration of primers and their generally shorter length), and the number of potential inter-primer interactions increases significantly with targeting ever increasing targets (multiplexing). Additionally, the amplification process itself introduces errors, and then current sequencing technologies have error rates in the 1-2%. Intrinsic error in the sequencing methodology can be overcome by the use of molecular identifiers (Mis) that can be incorporated in the initial stage of PCR amplification to "tag" and identify library molecules that correspond to a specific original molecule in the sample. Amplification and sequencing errors are removed by collapsing library molecules with the same MI to create a "consensus," removing spurious events that are not present among the entire MI family and keeping variants that are present. These "consensus reads" permit ultrasensitive detection of rare sequences - particularly valuable when detecting rare events as in cancer detection from cell-free DNA or samples with low tumor content. However, MI portions of oligos are inherently randomized sequences, which contribute to mis-priming during amplification. Mis-priming comes in the form of both primer dimers and amplification of non-targeted regions. The mis-priming not only results in lower amplification efficiency for targeted regions but also lower sequencing efficiency, as the amount of targeted product drops significantly.

Based on the issues described above, several technical challenges must be overcome to achieve a simple, single-tube approach for ultrasensitive multiplex library construction:
1. Avoid, or significantly reduce, non-specific interaction of multiplex primers.
2. Avoid, or significantly reduce, non-specific amplification of sequences, and/or primer dimers, due to random priming from MI barcodes.
3. Avoid, or significantly reduce, unintentional interactions between gene specific primers and universal primers during universal PCR amplification.

As used herein, the terms "sample" or "biological sample" (these terms are used interchangeably herein) encompass a variety of sample types obtained from a variety of sources, generally the sample types contain biological material. For example, the term includes biological samples obtained from a mammalian subject, e.g., a human subject, and biological samples obtained from a food, water, or other environmental source, etc. The definition encompasses blood and other liquid samples of biological origin, as well as solid tissue samples such as a biopsy specimen or tissue cultures or cells derived therefrom and the progeny thereof. The definition also includes samples that have been manipulated in any way after their procurement, such as by treatment with reagents, solubilization, or enrichment for certain components, such as polynucleotides. The term "Sample" encompasses a clinical sample, and also includes cells in culture, cell supematants, cell lysates, cells, serum, plasma, biological fluid, and tissue samples. "Sample" includes cells, e.g., bacterial cells or eukaryotic cells; biological fluids such as blood, cerebrospinal fluid, semen, saliva, and the like; bile; bone marrow; skin (e.g., skin biopsy); and antibodies obtained from an individual. Some non-limiting examples of a sample include liquid biopsy targets such as circulating cells (tumor, fetal, or stem), cellular components (e.g. nuclei), cell-free nucleic acids, extracellular vesicles, and protein antigens which are being targeted for development of non-invasive diagnostics for a variety of cancers. The term sample also includes biological targets indicative of disease such as prokaryotes, fungi, and viruses.

The term "DNA sample" as used herein encompass any DNA derived, synthesized, or reverse transcribed from a sample, or contained in a sample, and includes genomic DNA, cDNA, microdissected chromosome DNA, yeast artificial chromosome (YAC) DNA, cosmid DNA, phage DNA, Pl derived artificial chromosome (PAC) DNA, and bacterial artificial chromosome (BAC) DNA In some aspects, the DNA sample nucleic acids may have been manipulated using any method traditionally used in the art, such as, without limitation, restriction, ligation, or cloning.
In some aspects, the sample comprises mammalian DNA, plant DNA, yeast DNA, viral DNA, and prokaryotic DNA.
As described more fully herein, in various aspects the subject methods may be used to detect and amplify a variety of components from such biological samples. Components of interest include, but are not necessarily limited to, polynucleotides (e.g., DNA and/or RNA). Generally, the terms "targeted sequences", "target regions", and "target nucleic acid/s" are used interchangeably herein and encompasses any component of interest that may be present in a sample DNA, such as for example, specific region/s, or specific sequence/s, of the sample DNA

The terms "Primer/s", or "oligonucleotide/s", as used herein refer to linear polymers of nucleotide monomers, and may be used interchangeably. Primers, or oligonucleotides, can have any of a variety of structural configurations, e.g., be single stranded, double stranded, or a combination of both, as well as having higher order intra- or intermolecular secondary/tertiary structures, e.g., hairpins, loops, triple stranded regions, etc. The primers of the present disclosure typically range in size from a few monomeric units, e.g. 5 to two hundred monomeric units. In some aspects, the primers of the present disclosure typically range in size from fifty to one hundred monomeric units. Whenever a primer, or oligonucleotide, is represented by a sequence of letters (upper or lower case), such as "ATGCCTG," it will be understood that the nucleotides are in 5' to 3' order from left to right and that "A" denotes deoxyadenosine, "C" denotes deoxycytidine, "G" denotes deoxyguanosine, and "T" denotes deoxythymidine, "I" denotes deoxyinosine, "U" denotes deoxyuridine, unless otherwise indicated or obvious from context. Unless otherwise noted the terminology and atom numbering conventions will follow those disclosed in Strachan and Read, Human Molecular Genetics 2 (Wiley-Liss, New York, 1999).

Accordingly, the terms "nucleotide sequence", or "sequence", refer to is a succession of letters that indicate the order of nucleotides within linear polymers of DNA (using GACT), or RNA (GACU) molecules. The nucleotide, or nucleic acid, sequence is generally the primary structure of the DNA, or RNA, molecule.

### Primers design

The present invention discloses a targeted, multiplexed, amplification method for reproducible primer design. The presently disclosed amplification method employs two sets of primers, target- specific primers and universal primers. The target-specific primers (also referred to herein as" gene specific primers", or "hairpin primers") of the present invention comprise three critical components: a target-specific sequence to specifically hybridize with genetic sequences of choice, an adaptor sequence to allow for universal amplification, and a "lock" sequence which is comprises a portion, or all, of the target-specific sequence. Universal primers comprise an adaptor sequence that binds to the adaptor sequence of the target-specific amplification product and may also contain additional nucleotides, such as, but not limited to, sequences usable for indexing and sequencing the amplified DNA sequences (also referred to herein as amplification products or amplicons). In some some aspects, the presently disclosed amplification method is performed as a one-tube reaction, wherein all the amplification components, including the hairpin primers and universal primers, are present in the reaction mix and are utilized in sequential steps of the amplification reaction based on the method parameters, which will be further discussed below.

Generally, the hairpin primer nucleotide sequence, as described from the 3' end to the 5' end, may include some, or all, of the following nucleotide sequence elements:
a. Target-specific primer sequence: a sequence specific to the targeted region of the sample nucleic acids designed by one with an ordinary skill in the art of PCR primer design. The target- specific nucleotide sequence allows for specific base-pairing with the target nucleotide sequence.
b. Gene-specific hairpin de-stabilizer: an optional sequence comprising of at least one nucleotide that is non-complementary to the sequence upstream of the portion of the target nucleic acid sequence complementary to target-specific primer sequence to prevent the introduction of complementary bases from the MI during amplification.
c. Molecular Identifier (MI) which comprises an optional sequence of semi-random, or random, nucleotides.
d. Adaptor which comprises a nucleotide sequence which is used for hybridization with a universal primer sequence for the purpose of universal amplification (e.g., amplifying all, or most, of the nucleotide sequences which comprise one or more loci of a sequence complementary to the universal primer sequence).
e. Adaptor hairpin de-stabilizer: an optional sequence comprising of at least I nucleotide base derived from the gene-specific portion of the hairpin primer and that is non-complementary to the base(s) immediately 5' of the bases that are participating in the hairpin stem secondary structure of the gene-specific portion of the hairpin primer. Thus, preventing the adaptor sequence from participating in the hairpin stem structure.
f. Lock: a sequence of nucleotides that is complementary to a portion, or all, of the target- specific primer sequence portion of the hairpin primer. The lock sequence is able to form the hairpin stem structure by hybridizing with the target-specific primer sequence portion of the hairpin primer.
g. Stem de-stabilizer: an optional sequence comprising at least one nucleotide which is non- complementary to the 3' portion of the target-specific primer sequence portion of the hairpin pnmer.

An example of hairpin primers, designed to specifically target and amplify a portion of the GAPDH gene is depicted in Table 1. Table I depicts forward hairpin primer, GAPDH_9mer.F, and reverse hairpin primer, GAPDH_9mer.R. The target-specific primer sequence and the lock sequence are underlined. Gene-specific hairpin de-stabilizer sequences, adaptor hairpin de- stabilizer sequences, and stem de-stabilizer sequences are depicted in bold. The Molecular Identifier sequences and the adaptor sequences are depicted (these sequences are not underlined or in bold). The length of the target-specific primer sequence, and accordingly the lock sequence, is determined by the amount of sequence required to specifically target the nucleic acid of interest and the desired stability of the secondary hairpin structure. In some aspects, the length of the target-specific primer sequence is in the range of 5-30 nucleotides. In other aspects the length of the target-specific primer sequence is in the range of 10-20 nucleotides.
In yet other aspects the length of the target-specific primer sequence is 10, 11, 12, 13, 14, 15, 16, 17, 18, 19, or 20 nucleotides.

An example of the secondary hairpin structure of a hairpin primer is illustrated in FIG. 1. The hairpin stem is formed by complementary base-pairing of the target-specific primer sequence and the lock sequence, which comprise target-specific nucleotide sequences. The adenine-thymine base pairs are marked by blue dots, and the guanine-cytosine base pairs are marked by red dots. Gene-specific hairpin de-stabilizer sequences, A-G on the 5' end and G-A on the 3' end, are not part of the hairpin stem. The hairpin loop comprises some, or all, of the other sequence elements of the hairpin primer, such as gene-specific hairpin de-stabilizer, Molecular Identifier, adaptor, and/or Adaptor hairpin de-stabilizer.

Referring now to the universal primers, their nucleotide sequence, as described from the 3' end to the 5' end, may include some, or all, of the following elements:
a. Universal adaptor: the universal adaptor sequence comprises all, or a portion, of the adaptor sequence of the hairpin primer/s used in the same amplification reaction (e.g., PCR reaction).
b. Applications adaptor which is an oligonucleotide comprising nucleotide sequence usable for any applicable amplification reaction products, or genomic library, application. Such applications include, but are not limited to, indexing, barcoding, sequencing, attaching a tag for amplification products' detection, purification or quantification, or creating additional sequences for downstream applications such as next generation sequencing.

An example of universal primers, designed to amplify the amplification products (amplicons) of the hairpin primers of Table 1, is depicted in Table 1. Table 1 describes forward universal primer, Universal 500.F, and reverse universal primer, Universal 700.R. In the embodiment depicted in Table 1, Universal 500.F and Universal 700.R comprise universal primer sequences, and Illumina Paired End Adapters. The Paired End Adapters generally enable Illumina based applications, such as paired-end sequencing.

**Table 1, comprising embodiments of hairpin and universal primers, is depicted below:**

| Oligonucl eotide | Sequence (5' to 3') |
|---|---|
| GAPDH 9mer.F | AG TGCAAAAGGAGTGAGG GG ACACTCTTTCCCTACACGACGCTCTTCCGATCT WNNWNNWNN GA GCAGGGCCTCACTCCTTTTGCAGA (SEQ ID NO: 1) |
| GAPDH 9mer.R | CA ATGACAACGAATTTGGC AT GTGACTGGAGTTCAGACGTGTGCTCTTCCGATCT WNNWNNWNN TG CCTGTTGCTGTAGCCAAATTCGTTGTCATAC (SEQ ID NO: 2) |
| Universal 500.F | AATGATACGGCGACCACCGAGATCTACACNNNNNNNN ACACTCTTTCCCTACACGACGCTCTTCCGATCT (SEQ ID NO: 3) |
| Universal 700.R | CAAGCAGAAGACGGCATACGAGNNNNNNNNGTGTGACTGGAGTT CAGACGTGTGCTCTTCCGATCT (SEQ ID NO: 4) |

In the hairpin primers embodiment shown in Table 1, the MI nucleotide bases sequence is represented by the letter sequence , wherein each letter represents a nucleotide base in a semi-random 9mer. In this embodiment, N may be any nucleotide base, however positions 1, 4, and 7 (represented by the letter W) are restricted to either ATP or TTP. In other aspects, the MI nucleotide bases sequence is represented by the letter sequence NWNNWNNWN or letter sequence NNWNNWNNW, in these aspects, positions 2, 5 and 8 or positions 3, 6, and 9, respectively, are restricted to either ATP or TTP. The underlined bases in the said embodiment make up the hairpin stem structure, and the bases in between form the loop (as illustrated in FIG. 1). Referring now to the universal primers' embodiment depicted in Table 1, the italicized N's represent an 8mer nucleotide bases sequence, which may be used for example as an indexing barcode, however this portion of the universal primer is not limited to
the use mentioned above, and may be longer or shorter in length depending on its intended use. Indeed, universal primers with a shorter universal adaptor or a shorter indexing barcode sequence are contemplated herein. In the embodiment shown in Table 1, the applications adaptor sequences of the universal primers are PS and P7 flow cell sequence which are designed for sequencing on an Illumina instrument.

The term "universal primer sequence" generally refers to a primer binding site, e.g., a primer sequence that would be expected to hybridize (base-pair) to, and prime, one or more loci of complementary sequence, if present, on any nucleic acid fragment.

The "barcode" or "barcode sequence" as referred to herein, are polynucleotide sequences which are unique, i.e., distinguishable from other barcode sequences. The sequences may be of any suitable length which is sufficient to distinguish the barcode sequence from other barcode sequences. A barcode sequence may have a length of 5, 6, 7, 8, 9, 10, 11, 12, 13, 14, 15, 16, 17, 18, 19, 20, 21, 22, 23, 24, 25 nucleotides, or more. In some aspects, the barcodes are pre- defined and selected at random. The primers may be supplied by any oligonucleotide synthesis supplier.

### Hairpin primers and universal primers, and amplification method thereof

In some aspects, the hairpin and universal primers of the present invention are used for preparing a target specific library from sample nucleic acids using an amplification-based reaction. In some aspects, the amplification reaction is a one-tube reaction (single reaction). In some aspects, the amplification reaction is multiplexed (more than one target is selected). Amplification, as used herein, generally refers to methods for creating copies of nucleic acids by using thermal cycling to expose reactants to repeated cycles of heating and cooling, and to permit different temperature-dependent reactions (e.g. by Polymerase chain reaction (PCR)). As used herein, the sample nucleic acids may include any of a wide variety of nucleic acids, including, e.g., DNA and RNA, and specifically including for example, genomic DNA, cDNA, mRNA total RNA, and cDNA created from a mRNA or total RNA transcript. The sample nucleic acid may be derived, or prepared, using methods known in the art, from any one of the samples, or biological samples, of the instant disclosure.

In some aspects, the presently disclosed amplification reaction includes sample nucleic acids, amplification reagents, the hairpin primers of the instant disclosure, the universal primers of the instant disclosure, and a polymerase. The term "amplification reagents" encompass without limitation dNTPs (mix of the nucleotides dATP, dCTP, dGTP and dTTP), buffer/s, detergent/s, or solvent/s, as required. The polymerase used in the presently disclosed amplification reaction method is generally a DNA polymerase, and may be selected from, but is not limited to, Taq DNA polymerase, Phusion polymerase, or Q5 polymerase.
In some aspects, the amplification method includes initial amplification cycles designed for specific target amplification, and subsequent amplification cycles designed for uniform amplification of the amplification products from the said initial cycles. More specifically, the target-specific primer sequence annealing/extension temperature, universal adaptor annealing/extension temperature, and hairpin secondary structure denaturing temperature are designed such that the hairpin stem is open during the initial annealing and DNA synthesis (the term "DNA synthesis" is also referred to herein as "extension") cycles, but will be in the secondary hairpin structure in the subsequent amplification cycles, in which universal amplification takes place. In some aspects, the temperature difference between initial amplification cycles designed specifically for target amplification, and subsequent amplification cycles designed for uniform amplification of the amplification products from the said initial cycles, is in the range of about 0-20°C. In other aspects, the temperature difference between initial amplification cycles designed specifically for target amplification, and subsequent amplification cycles designed for uniform amplification of the amplification products from the said initial cycles, is in the range of about 5-l 5°C. In yet other aspects, the temperature difference between initial amplification cycles designed specifically for target amplification, and subsequent amplification cycles designed for uniform amplification of the amplification products from the said initial cycles, may be 5, 6, 7, 8, 9, 10, 11, 12, 13, 14, or 15°C. The precise temperature difference depends on the length of the gene-specific primer sequence and the universal adaptor sequence. In some aspects, the change of annealing/extension temperature described-above will occur during the transition DNA amplification cycles which follows the target specific DNA amplification cycles and precedes the universal DNA amplification cycles.

An embodiment of the present amplification method for one tube (one reaction) amplification of target nucleic acids and targeted sequencing library creation thereof is illustrated in FIG. 2. Thermal cycling with the target-specific hairpin primers and universal primers comprises the following steps:
A Gene-specific (also referred to herein as "target-specific") amplification: At least two cycles of annealing/extension are performed with a temperature which is at, or above, the denaturing temperature of the hairpin stem structure of the hairpin primers and within the annealing range of the gene-specific primer sequence. After two cycles, full amplicons are formed as a result of the amplification of the targeted sequences. Each amplicon contains the same hairpin structures as the hairpin primers which primed its synthesis. In some aspects, additional target-specific amplification cycles are performed to improve amplification efficiency. For example, 3, 4, 5, 6, 7, 8, 9, or 10 target-specific amplification cycles may be performed, as required.
B. Transition DNA amplification (in FIG. 2 marked "Transition"): In some aspects of the Transition DNA amplification step, at least two cycles of DNA amplification are performed at a lower annealing/extension temperature (or temperatures) than the gene-specific annealing/extension temperature used in step A, but higher than the universal amplification's annealing/extension temperature used in step C. In other aspects of the Transition DNA amplification step, at least two cycles of annealing/extension are performed at a temperature (or temperatures) which is in the range in between the gene-specific annealing/extension temperature used in step A, and the universal amplification's annealing/extension temperature used in step C. In any of the above-mentioned aspects of the Transition DNA amplification step, the annealing/extension temperature of the transition DNA amplification step may drop gradually with each cycle of annealing/extension. During the transition phase, the hairpin secondary structures are only partially open, which allows the universal adaptor sequence of the universal primers to bind to the adaptor sequence of the amplicons produced in the target-specific amplification step described in A The amplicons produced during the transition step will no longer contain the lock sequences derived from the hairpin primers' amplification. In some aspects, the amplicons produced at the transition step contain an applications adaptor. In the embodiment illustrated in FIG. 2, the amplicons contain applications adaptors which are the are Illumina sequencing adaptors i7 p7 and p5 i5. In some aspects, the transition DNA amplification step comprises 2-15 cycles of DNA amplification. In other aspects, the transition DNA amplification step comprises 2-10 cycles of DNA amplification. In yet other aspects, the transition DNA amplification step comprises 2, 3, 4, 5, 6, 7, 8, 9, or 10 cycles of DNA amplification.
C. Universal amplification: in some aspects of the universal amplification step, annealing/extension is performed at a temperature lower than the annealing/extension temperature of target-specific amplification step and the transition DNA amplification steps. In some aspects of the universal amplification step, annealing/extension is performed at a temperature equal to the temperature used during all, or some of, the transition DNA amplification's annealing/extension cycles. Therefore, the secondary hairpin structures of the hairpin primers and any amplicons from the target-specific amplification are generally maintained in a secondary structure form. The universal adaptor sequences, and optionally any applications adaptor sequences, of the universal primers are allowed to hybridize (base-pair) with complementary sequences of the amplicons produced in the transition phase of step B, and prime universal amplification of the said amplicons. The resulting amplification products (also referred to herein as "targeted sequencing library") as exemplified in FIG.2, are sequences of interest which contain at least an MI, universal adaptor sequence, and an application adaptor, and are ready for processing and analysis.
As used herein, DNA amplification comprises the steps of denaturing the DNA sample, annealing the hairpin primer with the DNA to allow the formation of a DNA-primer hybrid, and incubating the DNA-primer hybrid to allow the DNA polymerase to synthesize an amplification product. The steps of annealing the hairpin primer with the DNA to allow the formation of a DNA-primer hybrid, and incubating the DNA-primer hybrid to allow the DNA polymerase to synthesize an amplification product are generally referred to herein as "annealing/extension". Therefore, a certain number of DNA amplification cycles would mean the same number of annealing/extension cycles, and vice versa.

In some aspects, the annealing/extension temperature of the target-specific amplification step allows about 0-50% of the hairpin primers to retain their secondary hairpin structure. In other aspects, the annealing/extension temperature of the target-specific amplification step allows about 0-30% of the hairpin primers to retain their secondary hairpin structure. In yet other aspects, the annealing/extension of the target-specific amplification step allows about 0- 15% of the hairpin primers to retain their secondary hairpin structure. In yet other aspects, the annealing/extension temperature of the target-specific amplification step allows about 0%, 1%, 2%, 3%, 4%, 5%, 7%, 10%, 12%, or 15% of the hairpin primers to retain their secondary hairpin structure.

In some aspects, the annealing/extension temperature of the transition DNA amplification step allows about 50-100% of the hairpin primers to retain their secondary hairpin structure. In other aspects, the annealing/extension temperature of the transition DNA amplification step allows about 70-100% of the hairpin primers to retain their secondary hairpin structure. In yet other aspects, the annealing/extension temperature of the transition DNA amplification step allows about 85-100% of the hairpin primers to retain their secondary hairpin structure. In yet other aspects, the annealing/extension temperature of the transition DNA amplification step allows about 100%, 99%, 98%, 97%, 96%, 95%, 93%, 90%, or 85% of the hairpin primers to retain their secondary hairpin structure.

In some aspects, the annealing/extension temperature of the universal amplification step allows about 70-100% of the hairpin primers to retain their secondary hairpin structure. In other aspects, the annealing/extension temperature of the universal amplification step allows about 85-100% of the hairpin primers to retain their secondary hairpin structure. In yet other aspects, the annealing/extension temperature of the universal amplification step allows about 100%, 99%, 98%, 97%, 96%, 95%, 93%, 90%, or 85% of the hairpin primers to retain their secondary hairpin structure.

In some aspects, the presently described target nucleic acids amplification and targeted sequencing library creation thereof is multiplexed, e.g. more than one nucleic acid sequence is targeted. In the aspects wherein the target nucleic acids amplification method is multiplexed, a pair (forward and reverse) of target-specific hairpin primers is designed, as described above, and synthesized for each target of interest, wherein each of the said pairs may comprise unique identification sequences, such as Mis or barcodes sequences. In some aspects of multiplexed target-specific DNA amplification, the sequences of harpin primers of the reaction are generally designed so that the hairpin secondary structures thereof have similar denaturation temperatures. In some aspects of multiplexed target-specific DNA amplification, the universal primers are designed so that they can amplify all the amplicons from the gene-specific amplification step, or alternatively each pair of universal primer may amplify amplicons created by a specific pair of hairpin primers.

### PCR Methods

A feature of certain methods as described herein is the use of a polymerase chain reaction (PCR)- based assay to detect the presence of certain oligonucleotides and/or genes, e.g., oncogene(s) present in cells. Examples of PCR-based assays of interest include, but are not limited to, quantitative PCR (qPCR), quantitative fluorescent PCR (QF-PCR), multiplex fluorescent PCR (MF-PCR), digital droplet PCR (ddPCR) single cell PCR, PCR-RFLP/real time-PCR-RFLP, hot start PCR, nested PCR, in situ polony PCR, in situ rolling circle amplification (RCA), bridge PCR, picotiter PCR, emulsion PCR, and reverse transcriptase PCR (RT-PCR). Other suitable amplification methods include the ligase chain reaction (LCR), transcription amplification, self- sustained sequence replication, selective amplification of target polynucleotide sequences, consensus sequence primed polymerase chain reaction (CP-PCR), arbitrarily primed polymerase chain reaction (AP-PCR), degenerate oligonucleotide-primed PCR (DOP- PCR) and nucleic acid based sequence amplification (NAB SA).

A PCR-based assay may be used to detect the presence of certain gene(s), such as certain oncogene(s). In such assays, one or more primers specific to each gene of interest are reacted with the genome of each cell. These primers have sequences specific to the particular gene, so that they will only hybridize and initiate PCR when they are complementary to the genome of the cell. If the gene of interest is present and the primer is a match, many copies of the gene are created. To determine whether a particular gene is present, the PCR products may be detected through an assay probing the liquid of the monodisperse droplet, such as by staining the solution with an intercalating dye, like SybrGreen or ethidium bromide, hybridizing the PCR products to a solid substrate, such as a bead (e.g., magnetic or fluorescent beads, such as Luminex beads), or detecting them through an intermolecular reaction, such as FRET. These dyes, beads, and the like are each example of a "detection component," a term that is used broadly and generically herein to refer to any component that is used to detect the presence or absence of nucleic acid amplification products, e.g., PCR products.

### Amplification methods and amplification environments thereof

The methods disclosed herein can be performed in a variety of reaction volumes, wherein a volume for the purposes of the present invention is, generally, a volume in which reagents are entrained or otherwise releasably partitioned. In some aspects, the present amplification methods are particularly well-suited to nanoliter-sized water-in-oil partitioned reactions such as emulsions, microfluidically-generated droplets, or pre-templated instant partitions (PIPs). The above-mentioned small reaction volumes, in combination with loading of nucleic-acid targets based on Poisson statistics, results in the presence of O or I amplifiable targets in each reaction on average. Further, partitioned reactions reduce the amount of sample and assay reagents (such as dNTPs, buffer and polymerase) required for the amplification reaction, and increase the sensitivity of the assay as compared to conventional PCR assays.

The presently described methods are compatible with methods known in the art for preparation of partitions. Such methods include a variety of approaches such as, without limitation, shaking, vortexing, using microfluidic chips and/or associated devices, or the introduction of microscale particles (sometimes referred to as beads) that template the formation of uniform partitions (PIPs).

### Particles/beads and partitions

The methods of the present disclosure may be used with any suitable amplification environment including beads/particles, partitions, and combinations thereof.

In particular, beads/particles may provide a surface to which reagents are releasably attached, or a volume in which reagents are entrained or otherwise releasably partitioned. Non-limiting examples of such reagents include, e.g., enzymes, polypeptides, antibodies or antibody fragments, labeling reagents, e.g., dyes, fluorophores, chromophores, etc., nucleic acids, polynucleotides, oligonucleotides, and any combination of two or more of the foregoing. In some cases, the beads may provide a surface upon which to synthesize or attach oligonucleotide sequences. Various entities including oligonucleotides, barcode sequences, primers, crosslinkers and the like may be associated with the outer surface of a bead. In the case of porous beads, an entity may be associated with both the outer and inner surfaces of a bead. The entities may be attached directly to the surface of a bead (e.g., via a covalent bond, ionic bond, van der Waals interactions, etc.), may be attached to other oligonucleotide sequences attached to the surface of a bead (e.g. adaptor or primers), may be diffused throughout the interior of a bead and/or may be combined with a bead in a partition (e.g. fluidic droplet). In some aspects, the oligonucleotides (such as primers) are covalently attached to sites within the polymeric matrix of the bead and are therefore present within the interior and exterior of the bead. In some cases, an entity such as a cell or nucleic acid is encapsulated within a bead. Other entities including amplification reagents (e.g., PCR reagents, primers) may also be diffused throughout the bead or chemically-linked within the interior (e.g., via pores, covalent attachment to polymeric matrix) of a bead.

In some aspects, the particles are used to prepare significantly uniform reaction (such as amplification reaction according to the presently described methods) microenvironments. Particles, or beads, may be porous or nonporous. Particle may include microcompartments, which may contain additional components and/or reagents, e.g., additional components and/or reagents that may be releasable into monodisperse droplets.

### Example 1: Hairpin demonstration

In an embodiment of the present method, the hairpin secondary structure melt (denaturing) temperatures are 4°C higher than the gene-specific amplification step annealing/extension temperature. The target-specific primer sequences of the hairpin primers are designed to amplify a 57lbp portion of the GAPDH gene (the primers sequence is described in Table 1). Reactions are prepared using Ix Phusion GC buffer and Phusion polymerase, 0.2mM dNTP, I Ong human genomic DNA or nuclease-free water, 400nM GAPDH hairpin primers described in Table 1, and 3% DMSO or nuclease-free water. It is hypothesized, that facilitating the relaxation of the hairpin, the presence of DMSO would result in more efficient amplification than without its presence. Cycling (thermal cycling) is performed with all the cycles at the same annealing/extension temperature without the presence of universal primers: 98°C for 2 minutes followed by 30 cycles of 98°C for I minute and annealing/extension at either 50°C, 56°C, 62°C, 68°C, or 72°C for 2 minutes. After cycling, the reactions are diluted I :5 in water and loaded on a 2% agarose E-GEL EX gel (FIG. 3).

The hairpin primers are designed so that the Target-specific primer sequence portions of the primers are not available for amplification at lower annealing temperatures, but the hairpin structure denatures with an increase in temperature. In the presence of DMSO, the hairpin structure is more relaxed, resulting in more amplification product than in the reaction without DMSO. The hairpin structures in the hairpin primers used largely remain undenatured at temperatures below 72°C, even in the presence ofDMSO. At 72°C, the hairpin structure is denatured, and the target-specific primer sequences can amplify the target of interest.

### Example 2: Hairpin demonstration

In another embodiment of the experiment of Example 1, the same target-specific primer sequences portion of the hairpin primers were kept, however the lower hairpin secondary structure melt temperatures were designed by modifying the lock sequences. Amplification with the hairpin primers was performed using two different buffers provided by the enzyme (polymerase) manufacturer. Annealing/extension is performed at 55°C, 59°C, 63°C, 67°C, or 72°C for all cycles. Other than that, the design of the experiment is the same as described in Example 1. After completion of cycling, the reactions were diluted I :5 in water and loaded on a 2% agarose E-GEL EX gel (FIG. 4). As seen with the Example I set of GAPDH-specific primers, the hairpin lock denaturing temperatures can be tailored to the buffer system, and as also seen with Example I experiment, the locks can be fully engaged (maintain stem structure) below specific temperatures to impede amplification using the hairpin primers. Resulting in less product yield, Buffer 2 contains a different composition for PCR, causing the locks to be more engaged at 59°C than Buffer 1. At 67°C and 70°C, the locks are open (denatured) in both reactions (Buffer I and Buffer 2) to allow for similar amplification at both temperatures.

### Example 3: Singleplex Library Construction

With the addition ofMis, sequencing primers (such as universal primers), and sample indices (such as barcodes), the final expected library size for the GAPDH specific amplification products according to the method of Example I is 725bp. One-tube amplifications are performed using the hairpin and universal primers described in Table 1. Amplifications reactions are prepared with hairpin primers only or both universal and hairpin primers. For reactions with both sets of primers, reactions are prepared with 30ng of human genomic DNA or nuclease-free water for no-template controls (NTC). Reactions include the following components: Ix Fluent Biosciences high fidelity PCR buffer, GAPDH primers, universal primers, 0.2 mM dNTPs, DMSO, 0.5% Triton X-100, and Fluent Biosciences high fidelity polymerase. Cycling conditions are as follows: 98°C for 2 minutes, 2 cycles of 98°C for I minute and 72°C for 6 minutes, one cycle each of 98°C for I minute followed by 72°C, 70°C, 68°C, 66°C, 64°C, or 62°C for 3 minutes (a decrease by 2°C for each cycle for six cycles), and 30 cycles of 98°C for 30 seconds and 62°C for 90 seconds. After cycling, the reactions were diluted I :5 in water and loaded on an 2% agarose E-GEL EX for viewing via gel electrophoresis (FIG. 5). Generally, the 98°C for 2 minutes, 2 cycles of 98°C for I minute and 72°C for 6 minutes is an embodiment of the gene- specific phase, the one cycle each of 98°C for I minute followed by 72°C, 70°C, 68°C, 66°C, 64°C, or 62°C for 3 minutes (a decrease by 2°C for each cycle for six cycles) is an embodiment of the Transition phase, and the 30 cycles of 98°C for 30 seconds and 62°C for 90 seconds is an embodiment of the Universal amplification phase.

The cycling conditions are structured such that the hairpins structures are denatured at 72°C, allowing the gene-specific portions to amplify from the template (human genomic DNA). The products (amplicons) from the gene-specific phase amplification cycles contain hairpins; therefore, the amplicons' hairpins need to be partially open for the universal primers to prime them (annealing and allow DNA synthesis from the full amplicon to create the library). For that, not only do the hairpin structures need to be partially denatured, during the transition phase, but also the universal adaptors must be designed so to be able to anneal (base-pair), in the same temperature range. During the transition phase, the temperature is dropped gradually to fully close the hairpins, preventing the universal primers from binding to amplicons that include the hairpin structure and are therefore not the fully constructed library.

As mentioned above, to demonstrate constructing the library in a one-tube (one-reaction approach), PCR amplification was performed with and without the presence of the universal primers in the amplification reaction. In the first three lanes after the ladder (universal primers not included) in FIG. 5, no product is observed on the gel. The gene-specific primers are not able to engage (anneal) in later cycles (of the Transition and Universal amplification phases) due to the presence of the hairpin secondary structure, inhibiting amplification. In contrast, in the presence of universal primers (lanes 5-7), a product of the appropriate library size (725bp) is observed on the gel. To note, the addition of DMSO increases the yield, mainly by relaxing the secondary structure during the initial gene-specific cycles and again in the transition cycles when the universal primers engage.

### Example 4: Multiplex Demonstration

To demonstrate the present method with multiple targets, six pairs (a pair includes a forward primer and a reverse primer, designed to amplify a specific portion of nucleic acid sequence- for example, the primers GAPDH_9mer.F and GAPDH_9mer.R, both described in Table I) of hairpin primers were designed using the design requirements detailed in the present disclosure to generate six specific amplicons. Single-tube library amplifications reactions were performed using the following: Ix Fluent Biosciences high fidelity PCR buffer, hairpin primers (IDT), universal primers (IDT), 0.2 mM dNTPs (ThermoFisher, Cat# R0192), and Fluent Biosciences high fidelity polymerase. Cycling conditions were performed as follows: 98°C for 2 minutes, 2 cycles of 98°C for I minute and 72°C for 6 minutes, one cycle each of 98°C for I minute followed by 70°C, 68°C, 66°C, 64°C, or 62°C for 3 minutes, and 36 cycles of 98°C for 30 seconds and 62°C for 90 seconds.

Generally, the 98°C for 2 minutes, 2 cycles of 98°C for I minute and 72°C for 6 minutes is an embodiment of the gene-specific phase, the one cycle each of 98°C for I minute followed by 70°C, 68°C, 66°C, 64°C, or 62°C for 3 minutes is an embodiment of the Transition phase, and the 36 cycles of 98°C for 30 seconds and 62°C for 90 seconds is an embodiment of the Universal amplification phase.

To prepare libraries for sequencing, the amplification products are purified using a magnetic bead cleanup, and the libraries are quantified using fluorescence. After cleanup, the libraries are ready for sequencing. The overall process requires only one PCR amplification and cleanup, resulting in a process time of about four hours from extracted DNA to sequenceable library.

Without limiting the foregoing description, certain non-limiting aspects of the disclosure are provided below. As will be apparent to those of skill in the art upon reading this disclosure, each of the individually numbered aspects may be used or combined with any of the preceding or following individually numbered aspects. This is intended to provide support for all such combinations of aspects and is not limited to combinations of aspects explicitly provided below:
Aspects of the disclosure provide a method of library preparation. The method includes partitioning a mixture comprising a nucleic acid, a hairpin primer, and a polymerase into a plurality of partitions, wherein the hairpin primer comprises a hairpin structure that inhibits non-specific interactions with the hairpin primer; annealing, within one of the partitions, the hairpin primer to the nucleic acid; and performing an amplification reaction to extend the annealed hairpin primer with the polymerase, thereby creating an amplicon. The method may further include performing a second amplification reaction with a universal primer that includes a targeting sequence complementary to a portion of the amplicon. Preferably the partitions are aqueous droplets surrounded by oil within a tube. The partitioning, the amplification reaction, and the second amplification reaction may be performed within the tube and without lysing or releasing contents from the droplets. In some aspects, the partitioning is achieved by vortexing the tube. The mixture may further include a plurality of beads that template the formation of the droplets. In certain aspects, the amplification reaction is performed at a first temperature and the second amplification reaction is performed at a second temperature lower than the first temperature and lower than a third temperature at which the hairpin structure denatures. In certain aspects, the first temperature is in the range of about 50-70 degrees C and the second temperature is in the range of about 55-80 degrees C. In some aspects, the universal primer further comprises one or more of an indexing sequence, a barcode sequence, and a sequencing adaptor. The hairpin primer may include a molecular identifier sequence. In some aspects, the hairpin structure inhibits non-specific amplification of sequences by random priming via the molecular identifier sequence. The partitions may comprise pipetted emulsions or microfluidically-generated droplets. In some aspects, the mixture further comprises a universal primer, the universal primer comprising a molecular identifier sequence and a priming sequence that is complementary to a portion of the amplicon. Preferably the hairpin structure of the hairpin primer prevents non-specific priming via the molecular identifier sequence.

Aspects provide hairpin primer for amplifying a target nucleic acid or reverse complement thereof, the hairpin primer comprising: (a) a target-specific primer sequence comprising a nucleotide sequence complementary to a portion of the nucleotide sequence of the target nucleic acid; (b) an adaptor sequence; (c) a lock sequence comprising a sequence complementary to the said target-specific primer sequence; wherein sequences (a) to (c) are arranged from the 3' end to the 5' end of the said hairpin primer;
wherein the said target-specific primer sequence and complementary lock sequence are able to hybridize, thus allowing the said hairpin primer to form a secondary hairpin structure; and, wherein the target-specific primer sequence and lock sequence, when hybridized, form the stem portion of the said hairpin structure. The adapter sequence may comprises a universal primer sequence. The lock sequence may be complementary to a portion, or all, of the target-specific primer sequence. The hairpin primer may include a gene-specific hairpin de-stabilizer sequence located 5' to the target-specific primer sequence, wherein the gene-specific hairpin de- stabilizer sequence comprises of at least one nucleotide that is non-complementary to the sequence upstream of the portion of the target nucleic acid sequence complementary to target- specific primer sequence. The hairpin primer may further include a Molecular Identifier (MI) sequence located 5' to the gene-specific hairpin de-stabilizer sequence. The MI sequence may be a semi-random N-mer (N may be e.g., an integer between 4 and 20, preferably between 6 and 12); wherein the said Nmer optionally comprises three pre-determined sequence positions in which the nucleotide bases are restricted to either ATP or TTP; and, wherein the said pre-determined sequence positions are selected from sequence positions 1, 4, and 7, sequence positions 2, 5, and 8, or sequence positions 3, 6, and 9.

The hairpin primer may include an adaptor hairpin de-stabilizer sequence located 5' to the adaptor sequence, wherein the adaptor hairpin de-stabilizer sequence comprises at least one nucleotide derived from the gene-specific portion of the hairpin primer, wherein said one nucleotide is non-complementary to the first nucleotide of the hairpin primer that does not participate in the hairpin stem.

The hairpin primer may optionally include a stem de-stabilizer sequence located 5' to the lock sequence, wherein the stem de-stabilizer sequence comprises at least one nucleotide which is non-complementary to the 3' portion of the target-specific primer sequence of the hairpin primer. In some aspects, the secondary hairpin structure is denatured at a temperature in the range of about 55-80°C or the secondary hairpin structure is denatured at a temperature in the range of about 62-72°C, e.g., the secondary hairpin structure is denatured at a temperature of 62°C. Optionally, the secondary hairpin structure is denatured at a temperature of 72°C.

Aspects provide a method of amplifying target nucleic acid, the method comprising: (a)providing a single reaction mixture comprising:(i) DNA sample, (ii) a hairpin primer as described above, (iii) a universal primer comprising all, or some, of the adaptor sequence of the hairpin primer of (ii) (or reverse complement thereof), (iii) amplification reagents, and (iv)a DNA polymerase; (b) subjecting the sample DNA to DNA amplification wherein the hairpin primer anneals to target sequences to allow for production of a target-specific amplification product; and ( c) subjecting the target-specific amplification product of step (b) to DNA amplification wherein the universal primer anneals to the said amplification product to allow for universal amplification of the products of step (b). The DNA sample may be derived from a biological sample, e.g., genomic DNA. Preferably the DNA polymerase is selected from Taq DNA polymerase, Phusion polymerase, or Q polymerase. Optionally, the DNA amplification of step (b) is preceded by a DNA denaturing incubation. The DNA denaturing incubation may be performed at 98°C for two minutes. In certain aspects, the DNA amplification of step (b) comprises the steps of denaturing the DNA sample; annealing the hairpin primer with the DNA to allow the formation of a DNA-primer hybrid; and incubating the DNA-primer hybrid to allow the DNA polymerase to synthesize an amplification product. The DNA amplification of step (b) may be repeated at least two times. In some aspects, the annealing and DNA synthesis steps of the DNA amplification of step (b) are performed at a temperature in the range of about 55-80°C, in the range of about 62-72°C, or at a temperature of 62°C. It may be that the annealing and DNA synthesis steps of the DNA amplification of step (b) are performed at a temperature of 72°C. Optionally, the DNA amplification of step (c) comprises the steps of denaturing the DNA comprising amplification product of step (b); annealing the universal primer with the amplification product to allow the formation of a DNA-primer hybrid; and incubating the DNA-primer hybrid to allow the DNA polymerase to synthesize a second amplification product. In some aspects, the DNA amplification of step (c) is repeated at least twenty or even thirty times. In some aspects the annealing and DNA synthesis steps of the DNA amplification of step (c) are performed at a temperature lower than the temperature used for the annealing and DNA synthesis steps of step (b). In certain aspects the temperature used for the annealing and DNA synthesis steps of the DNA amplification of step (c) is lower than the temperature required for the denaturation of the hairpin secondary structure of the hairpin primer, thus preventing, or reducing the likelihood of, the hybridization of the hairpin primer to the amplification products of step (b), sample DNA, or the universal primer sequence of the universal primers. In some aspects, the annealing and DNA synthesis steps of the DNA amplification of step (c) are performed at a temperature in the range of about 50-80°C. Optionally the annealing and DNA synthesis steps of the DNA amplification of step (c) are performed at a temperature in the range of about 60-62°C, e.g., at a temperature of 62°C.

The method of amplifying target nucleic acid may further comprise a transition DNA amplification step performed after step (b) and before step (c).The transition DNA amplification step may include the steps of denaturing the DNA comprising the amplification product of step (b); annealing the universal primer or the hairpin primer with the said amplification product to allow the formation of a DNA-primer hybrid; and incubating the DNA-primer hybrid to allow the DNA polymerase to synthesize an amplification product. Optionally the transition DNA amplification step is repeated at least two times. Optionally the annealing and DNA synthesis steps of the transition DNA amplification step are performed at a temperature lower than the temperature used for the annealing and DNA synthesis steps of step (b), but higher than the temperature used for the annealing and DNA synthesis steps of step (c). Optionally the annealing and DNA synthesis steps of the transition DNA amplification step are performed at a temperature in the range between the temperature used for the annealing and DNA synthesis steps of step (b), and the temperature used for the annealing and DNA synthesis steps of step (c).

In some aspects, the annealing and DNA synthesis steps of the transition DNA amplification step are performed at a temperature in the range between the temperature used for the annealing and DNA synthesis steps of step (b), and the temperature used for the annealing and DNA synthesis steps of step (c), and wherein the temperature of annealing and DNA synthesis steps of the transition DNA amplification step drops gradually with every repeat. Preferably the likelihood of hybridization of the hairpin primer to the amplification products of step (b) is reduced with every repeat. In some aspects, the annealing and DNA synthesis steps of the transition DNA amplification step are performed at the first repeat at a temperature of 72°C, at a second repeat at a temperature of 70°C, at a third repeat at a temperature of 68°C, at a fourth repeat at a temperature of 66°C, at a fifth repeat at a temperature of 64°C, and at a sixth repeat at a temperature of 62°C. In some aspects the universal primer further comprises an application adaptor sequence. The application adaptor sequence may include an indexing sequence, a barcode sequence, a tag for amplification products' detection, purification or quantification, or a sequencing adaptor for sequencing applications. In some aspects, the universal primer for amplifying the amplification products is Universal 500.F, and the universal primer for amplifying the amplification products reverse complement is Universal 700.R. The amplification products may be used to prepare a sequencing library.

Aspects of the disclosure provide a composition for nucleic acid amplification. The composition includes a plurality of aqueous partitions. One of the partitions comprises: a bead; a hairpin primer comprising a stem and loop structure that inhibits non-specific hybridization; a target nucleic acid; and a polymerase. The partitions may comprise aqueous droplets formed and contained within a tube. The droplets may be formed by vortexing the tube. The bead may template the formation of one of the droplets. Preferably the one partition further comprises a universal primer that includes a molecular identifier sequence and priming sequence that is complementary to an amplicon created by extending the hairpin primer annealed to the target nucleic acid. Preferably the stem and loop structure of the hairpin primer prevents non-specific priming via the molecular identifier sequence.

## Claims

1. A method of library preparation, the method comprising:
partitioning a mixture comprising a nucleic acid, a hairpin primer, and a polymerase into a plurality of partitions, wherein the hairpin primer comprises a hairpin structure that inhibits non-specific interactions with the hairpin primer;
annealing, within one of the partitions, the hairpin primer to the nucleic acid; and performing an amplification reaction to extend the annealed hairpin primer with the polymerase, thereby creating an amplicon; and
performing a second amplification reaction with a universal primer that includes a targeting sequence complementary to a portion of the amplicon;
wherein the amplification reaction is performed at a first temperature and the second amplification reaction is performed at a second temperature lower than the first temperature and lower than a third temperature at which the hairpin structure denatures.

2. The method of claim 1, wherein the partitions are aqueous droplets surrounded by oil within a tube.

3. The method of claim 2, wherein the partitioning, the amplification reaction, and the second amplification reaction are performed within the tube and without lysing or releasing contents from the droplets.

4. The method of claim 2, wherein partitioning is achieved by vortexing the tube.

5. The method of claim 2, wherein the mixture further comprises a plurality of beads that template the formation of the droplets.

6. The method of claim 1, wherein the first temperature is in the range of about 50-70 degrees C and the second temperature is in the range of about 55-80 degrees C.

7. The method of claim 1, wherein the universal primer further comprises one or more of an indexing sequence, a barcode sequence, and a sequencing adaptor.

8. The method of claim 1, wherein the hairpin primer comprises a molecular identifier sequence; and optionally wherein the hairpin structure inhibits non-specific amplification of sequences by random priming via the molecular identifier sequence.

9. The method of claim 1, wherein the partitions comprise pipetted emulsions or microfluidically-generated droplets.

10. The method of claim 1, wherein the mixture further comprises a universal primer, the universal primer comprising a molecular identifier sequence and a priming sequence that is complementary to a portion of the amplicon.

11. The method of claim 10, wherein the hairpin structure of the hairpin primer prevents non specific priming via the molecular identifier sequence.

12. A composition suitable for nucleic acid amplification by the method of claim 1, the composition comprising:
a plurality of aqueous partitions, wherein one of the partitions comprises:
a bead;
a hairpin primer comprising a stem and loop structure that inhibits non-specific hybridization;
a target nucleic acid;
a polymerase; and
a universal primer that includes a priming sequence that is complementary to an amplicon created by extending the hairpin primer annealed to the target nucleic acid, wherein the partitions comprise aqueous droplets formed by vortexing; and wherein the bead templates the formation of one of the droplets.

13. The composition of claim 12, wherein the universal primer further includes a molecular identifier sequence.

14. The composition of claim 13, wherein the stem and loop structure of the hairpin primer prevents non-specific priming via the molecular identifier sequence.

## Patentansprüche

1. Verfahren für eine Bibliothekerstellung, das Verfahren umfassend:
Partitionieren einer Mischung, umfassend eine Nukleinsäure, einen Haarnadelprimer und eine Polymerase, in eine Vielzahl von Partitionierungen, wobei der Haarnadelprimer eine Haarnadelstruktur umfasst, die nichtspezifische Wechselwirkungen mit dem Haarnadelprimer hemmt;
Aufschmelzen, innerhalb einer der Partitionierungen, des Haarnadelprimers an die Nukleinsäure; und Durchführen einer Amplifikationsreaktion, um den aufgeschmolzenen Haarnadelprimer mit der Polymerase zu verlängern, wodurch ein Amplikon erzeugt wird; und
Durchführen einer zweiten Amplifikationsreaktion mit einem Universalprimer, der eine Zielsequenz einschließt, die zu einem Abschnitt des Amplikons komplementär ist;
wobei die Amplifikationsreaktion bei einer ersten Temperatur durchgeführt wird und die zweite Amplifikationsreaktion bei einer zweiten Temperatur durchgeführt wird, die niedriger als die erste Temperatur und niedriger als eine dritte Temperatur ist, bei der die Haarnadelstruktur denaturiert.

2. Verfahren nach Anspruch 1, wobei die Partitionierungen wässrige Tropfen sind, die innerhalb eines Rohrs durch Öl umgeben sind.

3. Verfahren nach Anspruch 2, wobei das Partitionieren, die Amplifikationsreaktion und die zweite Amplifikationsreaktion innerhalb des Rohrs und ohne ein Lysieren oder Freisetzen von Inhalten aus den Tropfen durchgeführt werden.

4. Verfahren nach Anspruch 2, wobei das Partitionieren durch ein Verwirbeln des Rohrs erreicht wird.

5. Verfahren nach Anspruch 2, wobei die Mischung ferner eine Vielzahl von Perlen umfasst, die als Vorlage für die Ausbildung der Tropfen dienen.

6. Verfahren nach Anspruch 1, wobei die erste Temperatur in dem Bereich von etwa 50-70 Grad C liegt und die zweite Temperatur in dem Bereich von etwa 55-80 Grad C liegt.

7. Verfahren nach Anspruch 1, wobei der Universalprimer ferner eines oder mehrere umfasst von einer Indexierungssequenz, einer Barcodesequenz und einem Sequenzierungsadapter.

8. Verfahren nach Anspruch 1, wobei der Haarnadelprimer eine molekulare Identifikationssequenz umfasst; und wobei optional die Haarnadelstruktur eine nichtspezifische Amplifikation von Sequenzen durch zufälliges Priming über die molekulare Identifikationssequenz hemmt.

9. Verfahren nach Anspruch 1, wobei die Partitionierungen pipettierte Emulsionen oder mikrofluidisch generierte Tropfen umfassen.

10. Verfahren nach Anspruch 1, wobei die Mischung ferner einen Universalprimer umfasst, der Universalprimer umfassend eine molekulare Identifizierungssequenz und eine Primingsequenz, die zu einem Abschnitt des Amplikons komplementär ist.

11. Verfahren nach Anspruch 10, wobei die Haarnadelstruktur des Haarnadelprimers ein nichtspezifisches Priming über die molekulare Identifizierungssequenz verhindert.

12. Zusammensetzung, die für die Nukleinsäureamplifikation durch das Verfahren nach Anspruch 1 geeignet ist, die Zusammensetzung umfassend:
eine Vielzahl von wässrigen Partitionierungen, wobei eine der Partitionierungen umfasst:
eine Perle;
ein Haarnadelprimer, umfassend eine Stamm- und Schleifenstruktur, die eine nichtspezifische Hybridisierung hemmt;
eine Zielnukleinsäure;
eine Polymerase; und
einen Universalprimer, der eine Primingsequenz einschließt, die komplementär zu einem Amplikon ist, das durch das Verlängern des Haarnadelprimers erzeugt wird, der an die Zielnukleinsäure aufgeschmolzen ist, wobei die Partitionierungen wässrige Tropfen umfassen, die durch Verwirbeln ausgebildet sind; und wobei die Perle als Vorlage für die Ausbildung der Tropfen dient.

13. Zusammensetzung nach Anspruch 12, wobei der Universalprimer ferner eine molekulare Identifikationssequenz einschließt.

14. Zusammensetzung nach Anspruch 13, wobei die Stamm- und Schleifenstruktur des Haarnadelprimers ein nichtspezifisches Priming über die molekulare Identifizierungssequenz verhindert.

## Revendications

1. Procédé de préparation de banque, le procédé comprenant :
le partitionnement d'un mélange comprenant un acide nucléique, une amorce en épingle à cheveux et une polymérase en plusieurs partitions, dans lequel l'amorce en épingle à cheveux comprend une structure en épingle à cheveux qui inhibe les interactions non spécifiques avec l'amorce en épingle à cheveux ;
le recuit, dans l'une des partitions, de l'amorce en épingle à cheveux sur l'acide nucléique ; et le fait d'effectuer une réaction d'amplification pour étendre l'amorce en épingle à cheveux recuite avec la polymérase, créant ainsi un amplicon ; et
le fait d'effectuer une seconde réaction d'amplification avec une amorce universelle qui comporte une séquence de ciblage complémentaire d'une partie de l'amplicon ;
dans lequel la réaction d'amplification est effectuée à une première température et la seconde réaction d'amplification est effectuée à une deuxième température inférieure à la première température et inférieure à une troisième température à laquelle la structure en épingle à cheveux se dénature.

2. Procédé selon la revendication 1, dans lequel les partitions sont des gouttelettes aqueuses entourées d'huile à l'intérieur d'un tube.

3. Procédé selon la revendication 2, dans lequel le partitionnement, la réaction d'amplification et la seconde réaction d'amplification sont effectués à l'intérieur du tube et sans lyser ou libérer le contenu des gouttelettes.

4. Procédé selon la revendication 2, dans lequel le partitionnement est obtenu par vortexage du tube.

5. Procédé selon la revendication 2, dans lequel le mélange comprend en outre une pluralité de perles qui modèlent la formation des gouttelettes.

6. Procédé selon la revendication 1, dans lequel la première température est comprise entre 50 et 70 degrés C et la deuxième température est comprise entre 55 et 80 degrés C.

7. Procédé selon la revendication 1, dans lequel l'amorce universelle comprend en outre une ou plusieurs séquences d'indexation, une séquence de code-barres et un adaptateur de séquençage.

8. Procédé selon la revendication 1, dans lequel l'amorce en épingle à cheveux comprend une séquence d'identification moléculaire ; et éventuellement dans lequel la structure en épingle à cheveux inhibe l'amplification non spécifique des séquences par amorçage aléatoire par l'intermédiaire de la séquence d'identification moléculaire.

9. Procédé selon la revendication 1, dans lequel les partitions comprennent des émulsions pipetées ou des gouttelettes générées par microfluidique.

10. Procédé selon la revendication 1, dans lequel le mélange comprend en outre une amorce universelle, l'amorce universelle comprenant une séquence d'identification moléculaire et une séquence d'amorçage qui est complémentaire d'une partie de l'amplicon.

11. Procédé selon la revendication 10, dans lequel la structure en épingle à cheveux de l'amorce en épingle à cheveux empêche l'amorçage non spécifique par l'intermédiaire de la séquence d'identification moléculaire.

12. Composition convenant à l'amplification d'acides nucléiques par le procédé selon la revendication 1, la composition comprenant :
une pluralité de partitions aqueuses, dans lequel l'une des partitions comprend :
une perle ;
une amorce en épingle à cheveux comprenant une tige et une structure en boucle qui inhibe l'hybridation non spécifique ;
un acide nucléique cible ;
une polymérase ; et
une amorce universelle qui comporte une séquence d'amorçage complémentaire d'un amplicon créé par l'extension de l'amorce en épingle à cheveux recuit sur l'acide nucléique cible, dans laquelle les partitions comprennent des gouttelettes aqueuses formées par vortexage ; et dans laquelle la perle modèle la formation de l'une des gouttelettes.

13. Composition selon la revendication 12, dans laquelle l'amorce universelle comporte en outre une séquence d'identification moléculaire.

14. Composition selon la revendication 13, dans laquelle la structure en tige et en boucle de l'amorce en épingle à cheveux empêche l'amorçage non spécifique par la séquence d'identification moléculaire.
